(19) Europäisches Patentamt
European Patent Office
Office européen des brevets

(11) Veröffentlichungsnummer: **0 322 759 B1**

(12) **EUROPÄISCHE PATENTSCHRIFT**

(45) Veröffentlichungstag der Patentschrift: **01.09.93**

(51) Int. Cl.5: **C07C 271/08**, C07C 271/40, C07C 275/28, C07C 311/03, C07C 275/62, D06M 13/342

(21) Anmeldenummer: **88121483.7**

(22) Anmeldetag: **22.12.88**

# (54) Urethane aus aliphatischen Fluoralkoholen, Isocyanaten und substituierten aromatischen Verbindungen, Verfahren zu ihrer Herstellung und ihre Verwendung.

(30) Priorität: **29.12.87 DE 3744423**

(43) Veröffentlichungstag der Anmeldung:
**05.07.89 Patentblatt 89/27**

(45) Bekanntmachung des Hinweises auf die Patenterteilung:
**01.09.93 Patentblatt 93/35**

(84) Benannte Vertragsstaaten:
**AT BE CH DE FR GB IT LI NL**

(56) Entgegenhaltungen:
**EP-A- 0 160 402**
**EP-A- 0 253 186**

**CHEMICAL ABSTRACTS, Band 90, Nr. 11, 1979, Seite 569, Zusammenfassung Nr. 86861z, Columbus, Ohio, USA; H. MATSUO et al: "Alicyclic fluoroalkyl urethanes"; & JP-A-78 112 855**

**CHEMICAL ABSTRACTS, Band 76, Nr. 8, 21. Februar 1972, Seite 42, Zusammenfassung Nr. 35213t, Columbus, Ohio, USA; K. TERAMURA et al: "Water- and oil-repellent treatment of fibers"; & JP-A-71 00348**

(73) Patentinhaber: **HOECHST AKTIENGESELLSCHAFT**

**D-65926 Frankfurt(DE)**

(72) Erfinder: **Wehowsky, Franz, Dr.**
**Talhauser Strasse 27**
**D-8269 Burgkirchen(DE)**
Erfinder: **Kleber, Rolf, Dr.**
**Am Trieb 41**
**D-6078 Neu-Isenburg(DE)**
Erfinder: **Jaeckel, Lothar**
**Kettelerstrasse 88**
**D-6093 Flörsheim am Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft Urethane, die aus aliphatischen Fluoralkoholen, Isocyanaten und substituierten aromatischen Verbindungen zusammengesetzt sind. Die Erfindung betrifft ferner ein Verfahren zur Herstellung dieser Urethane und ihre Verwendung.

Aus der europäischen Patentanmeldung 0 172 717-A2 ist es bekannt, Urethane aus mindestens einem aliphatischen Fluoralkohol mit einer Perfluoralkylgruppe als Fluorkomponente und einem Tris-(isocyanatoalkan)biuret als Isocyanatkomponente durch Inkorporierung einer modifizierenden Gruppe in den Eigenschaften bezüglich Textilausrüstung zu verbessern. Die modifizierende Gruppe kann eine aromatische, aliphatische, alicyclische Verbindung oder eine Mischung von solchen Verbindungen mit einem oder mehreren aktiven Wasserstoffatomen sein (über die aktiven Wasserstoffatome erfolgt die Verknüpfung der Verbindungen mit Isocyanatgruppen). Von den genannten Modifiern werden im wesentlichen nur aliphatische Verbindungen näher beschrieben.

Es sind auch schon Urethane zur Ausrüstung von Textilien vorgeschlagen worden, die im Molekül mindestens eine Perfluoralkylgruppe in Form eines aliphatischen Fluoralkohols, Isocyanat und gegebenenfalls mindestens eine Epichlorhydringruppe sowie mindestens eine durch ein aktives Wasserstoffatom addierte aromatische Dihydroxy-, Diamino-, Aminohydroxy-, Aminocarboxy- oder eine aromatische Hydroxycarboxy-Verbindung enthalten, wobei diese aromatischen Verbindungen die modifizierenden Komponenten darstellen (vergleiche europäische Patentanmeldung 0 253 186-A2).Diese Urethane weisen sehr gute Eigenschaften im Hinblick auf die Ausrüstung von Textilien auf.

Urethane aus aliphatischen Fluoralkoholen, Isocyanaten und phenolartigen modifizierenden Komponenten sind auch in C.A. 90, 86861z (1979) und C.A. 76, 35213t (1972) beschrieben. Die Isocyanatverbindung ist dabei immer ein Diisocyanat. Andere als geeignet erachtete Isocyanate werden nicht erwähnt, noch wird darauf hingewiesen.

Es wurden nun weitere Urethane aus aliphatischen Fluoralkoholen, Isocyanaten und aromatischen Verbindungen gefunden, die hervorragende Textilausrüstungsmittel darstellen. Es wurde nämlich gefunden, daß spezielle substituierte Phenol- und Anilinderivate besonders wirksame modifizierende Komponenten darstellen, wenn sie unter anderem mit einem bestimmten Triisocyanat kombiniert sind. Die neuen Urethan-Verbindungen sind demnach zusammengesetzt aus mindestens einem aliphatischen Fluoralkohol, Triisocyanat and gegebenenfalls mindestens einer Epichlorhydringruppe sowie mindestens einer durch ein aktives Wasserstoffatom addierten aliphatisch oder aromatisch substituierten Phenol- oder Anilin-Verbindung.

Die erfindungsgemäßen Urethane entsprechen der nachstehenden allgemeinen Formel 1

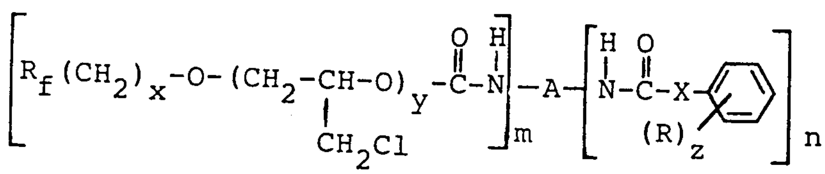

(1),

worin bedeuten:

- $R_f$ eine Perfluoralkylgruppe mit 4 bis 20 C-Atomen, vorzugsweise 6 bis 16 C-Atomen, oder eine $R_f'SO_2NR^1$-Gruppe,in der $R_f'$ eine Perfluoralkylgruppe mit 4 bis 20 C-Atomen ist, vorzugsweise mit 6 bis 16 C-Atomen, und $R^1$ H oder eine Alkylgruppe mit 1 bis 4 C-Atomen ist,
- x eine ganze Zahl von 1 bis 4, vorzugsweise 2,
- y eine Zahl von 0 bis 10, vorzugsweise 1 bis 5,
- m eine Zahl von 1 bis 2 und
- n eine Zahl von 1 bis 2, wobei die Summe aus m + n höchstens 3 ist,
- A eine Gruppe entsprechend den nachstehenden Formeln 2 und 3 (das sind isocyanatfreie Reste), vorzugsweise der Formel 3

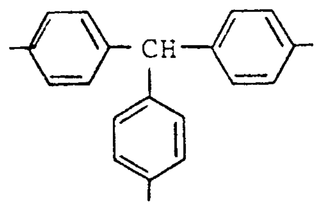 (2)

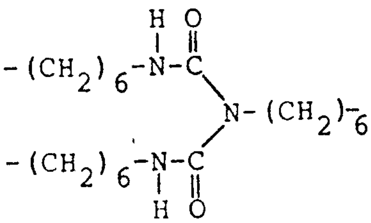 (3)

X -O- oder -NH-,

z 1, 2 oder 3, vorzugsweise 1 oder 2, und

R eine Alkylgruppe mit 1 bis 18 C-Atomen, vorzugsweise 1 bis 12 C-Atomen, eine Alkenylgruppe mit 2 bis 18 C-Atomen, vorzugsweise 2 bis 12 C-Atomen, eine Arylgruppe oder eine Alkarylgruppe, wobei R auch mehrere dieser Bedeutungen haben kann.

Von den angegebenen Bedeutungen für $R_f$ ist die Perfluoralkylgruppe mit 4 bis 20 C-Atomen, vorzugsweise 6 bis 16 C-Atomen, bevorzugt. Die Perfluoralkylgruppe kann geradkettig oder verzweigt sein; im Falle einer verzweigten Perfluoralkylgruppe ist die endverzweigte bevorzugt. Von den beiden Perfluoralkylgruppen, den geradkettigen oder verzweigten, sind die geradkettigen bevorzugt. Beim Perfluoralkyl-Rest handelt es sich in der Regel um ein Gemisch von Perfluoralkylgruppen mit der obengenannten Anzahl von C-Atomen. A ist, wie bereits erwähnt vorzugsweise der Triisocyanatrest gemäß Formel 3. Zu den Bedeutungen von R wird im einzelnen noch folgendes ausgeführt: die Alkyl- und die Alkenylgruppen können geradkettig oder verzweigt sein, die Alkenylgruppen haben vorzugsweise höchstens drei Doppelbindungen. Die Arylgruppe kann ein- oder mehrfach, vorzugsweise ein- bis dreifach substituiert sein. Bevorzugte Vertreter von R als Arylgruppe sind Phenyl oder Phenyl, ein- bis dreifach substituiert mit $C_1$ bis $C_6$-Alkyl. Die Alkarylgruppe besteht vorzugsweise aus einer Alkylgruppe mit vorzugsweise 1 bis 4 C-Atomen, die ein- bis dreifach substituiert ist mit Phenyl oder Phenyl, ein- bis dreifach substituiert mit $C_1$ bis $C_6$-Alkyl, oder mit 2 bis 3 Hydroxyphenylgruppen, die jeweils ein- bis zweifach mit $C_1$ bis $C_6$-Alkyl substituiert sein können (das genannte $C_1$ bis $C_4$-Alkyl und $C_1$ bis $C_6$-Alkyl kann geradkettig oder verzweigt sein).

Die Herstellung der erfindungsgemäßen Urethane ergibt sich aus der allgemeinen Formel 1 und wird im folgenden näher beschrieben. Sie werden hergestellt durch Reaktion eines aliphatischen Fluoralkohols der Formel

$$R_f(CH_2)_xO(CH_2\underset{\substack{|\\ CH_2Cl}}{C}HO)_y{-}H \quad ,$$

worin $R_f$, x und y die obengenannte Bedeutung haben,
mit einem Triisocyanat entsprechend einer der Gruppen der Formeln 2 und 3 zum Addukt der Formel

$$\left[R_f(CH_2)_xO(CH_2\underset{\substack{|\\ CH_2Cl}}{C}HO)_y{-}CONH\right]_m{-}A{-}(NCO)_n \qquad \text{(Addukt 1)},$$

worin $R_f$, x, y, m, n und A die obengenannte Bedeutung haben,
und durch Reaktion dieses Adduktes mit einem substituierten Phenol oder Anilin der nachstehenden Formel

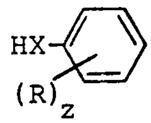

,

worin X, R und z die genannten Bedeutungen haben,
zu den erfindungsgemäßen Urethanen der angegebenen Formel 1.

Im folgenden wird die Herstellung der erfindungsgemäßen Verbindungen im einzelnen beschrieben.
Zur Herstellung des Adduktes 1 werden aliphatische Fluoralkohole mit einer Perfluoralkyl-Gruppe in Form eines Perfluorhydroalkanols oder Perfluorsulfonamidoalkanols und gegebenenfalls mit mindestens einer Epichlorhydrin-Gruppe (entsprechend der Bedeutung von y in Formel 1) eingesetzt. Perfluorhydroalkanole und Perfluorsulfonamidoalkanole, wie sie zur Herstellung des Adduktes 1 eingesetzt werden, wenn in Formel 1 y = Null ist, sind schon seit langem bekannt und brauchen deshalb nicht mehr näher beschrieben zu werden. Die aliphatischen Fluoralkohole mit einer Perfluoralkyl-Gruppe und mit Epichlorhydrin-Gruppen werden dadurch erhalten, daß man beispielsweise Perfluoralkylethanol (als Perfluorhydroalkanol) oder beispielsweise Perfluoralkylsulfonamidoethanol (als Perfluorsulfonamidoalkanol) mit Epichlorhydrin (Siedepunkt bei Normalbedingungen 116 °C), gegebenenfalls in Gegenwart von Lewis-Säuren als Katalysator, bei einer Temperatur von 30 bis 100 °C, vorzugsweise 40 bis 70 °C, umsetzt, wobei die Ethanolverbindung und das Epichlorhydrin im Molverhältnis von etwa 1 : y eingesetzt werden (y hat die in Rede stehende Bedeutung). Bei Perfluorhydroalkanol und beim Perfluorsulfonamidoalkanol handelt es sich bezüglich Perfluoralkyl-Rest in der Regel um preisgünstige, im Handel erhältliche Gemische mit im wesentlichen 6 bis 20 C-Atomen. Die Art der Lewis-Säure ist nicht kritisch. Bevorzugt sind $BF_3$, Bortrifluoriddiethyletherat, $SnCl_4$, $SbCl_5$, $TiCl_4$, $FeCl_3$, $PF_5$ und/oder Dibutylzinndilaurat, wobei Bortrifluoriddiethyletherat besonders bevorzugt ist. Die Menge an Katalysator beträgt im allgemeinen 0,01 bis 5 Gew.-%, vorzugswiese 0,1 bis 1 Gew.-%, bezogen auf Perfluoralkylethanol. Die Umsetzung wird vorzugsweise unter Rühren und bei dem sich einstellenden Druck durchgeführt. Die Umsetzungsdauer liegt im Bereich von etwa 0,5 bis 7 h. Es kann zweckmäßig sein, ein Lösungsmittel einzusetzen. Bevorzugte Lösungsmittel sind halogenierte Kohlenwasserstoffe wie Tetrachlorkohlenstoff, Trichlorethylen, 1,2-Dichlorethan, Trichlorethan und Trifluortrichlorethan; Ketone wie Methylethylketon und Cyclohexanon; Ether wie Diisopropylether und Tetrahydrofuran. Die in Rede stehende Umsetzung läuft quantitativ ab. Im erhaltenen Reaktionsprodukt wird das gegebenenfalls verwendete Lösungsmittel abdestilliert, wobei auch gegebenenfalls vorhandene flüchtige Anteile wie nichtumgesetztes Epichlorhydrin entfernt werden. Aus Zweckmäßigkeitsgründen kann man die Destillation auch unter Vakuum (Wasserstrahl-Vakuum) durchführen. Die als Katalysator eingesetzte Lewis-Säure, die bei der nachfolgenden Umsetzung mit Isocyanat an sich nicht stört, kann mit Hilfe von alkalischen Mitteln, vorzugsweise mit Hilfe einer wäßrigen Natriumbicarbonat-Lösung oder einem Amin wie Triethylamin, ausgewaschen beziehungsweise neutralisiert werden. Der aliphatische Fluoralkohol mit einer Perfluoralkyl-Gruppe und mit Epichlorhydrin-Gruppen stellt ein wachsartiges, gelbgefärbtes Produkt dar.

Zur Herstellung des Adduktes 1 wird vorzugsweise so vorgegangen, daß man einen aliphatischen Fluoralkohol mit einer Perfluoralkyl-Gruppe und gegebenenfalls mit mindestens einer Epichlorhydrin-Gruppe (entsprechend der Bedeutung von y in Formel 1) mit einem Isocyanat entsprechend den Formeln 2 oder 3 bei einer Temperatur von 70 bis 150 °C, vorzugsweise 90 bis 130 °C, umsetzt, wobei der aliphatische Fluoralkohol und das Isocyanat in dem Molverhältnis eingesetzt werden, das sich aus der angestrebten Bedeutung für m und n in der Formel für das Addukt 1 ergibt. Die Umsetzung wird vorzugsweise unter Rühren und bei dem sich einstellenden Druck durchgeführt und - sofern es zweckmäßig ist, beispielsweise zur Verkürzung der Reaktionszeit - in Gegenwart der obengenannten Lewis-Säure-Katalysatoren. Es können auch Lösungsmittel, zum Beispiel Ester, eingesetzt werden. Die Umsetzungsdauer liegt im Bereich von 1 bis 15 h. Bei dem Isocyanat wird es sich oft um im Handel erhältliche Isocyanat-Gemische handeln. Die Umsetzung des in Rede stehenden aliphatischen Fluoralkohols mit Isocyanat zum Addukt 1 verläuft quantitativ. Die erhaltenen Produkte können gegebenenfalls gereinigt werden, beispielsweise können flüchtige Anteile abdestilliert werden. Das Addukt 1 stellt ein wachsartiges, gelbgefärbtes Produkt dar.

Zur Herstellung der erfindungsgemäßen Urethane der Formel 1 wird vorzugsweise so vorgegangen, daß man das Addukt 1 mit einer Phenol- oder Anilin-Verbindung der angegebenen Formel bei einer Temperatur von 70 bis 150 °C, vorzugsweise 90 bis 130 °C, umsetzt, wobei die Addukt-1-Verbindung und die Phenol- oder Anilin-Verbindung in einem solchen Molverhältnis eingesetzt werden, daß die Molmenge an Phenol-

oder Anilin-Verbindung den in dem eingesetzten Addukt 1 vorhandenen freien Isocyanat-Gruppen entspricht. Die Umsetzung wird vorzugsweise unter Rühren und bei dem sich einstellenden Druck durchgeführt und - sofern es zweckmäßig ist, beispielsweise zur Verkürzung der Reaktionszeit - in Gegenwart der obengenannten Lewis-Säure-Katalysatoren. Es können auch Lösungsmittel, zum Beispiel Ester, eingesetzt werden. Die Umsetzungsdauer liegt im Bereich von 1 bis 40 h. Die Umsetzung des Adduktes 1 mit der Phenol- oder Anilin-Verbindung zu den erfindungsgemäßen Urethanen der Formel 1 verläuft quantitativ. Das erhaltene Urethan kann gegebenenfalls gereinigt werden, beispielsweise können flüchtige Anteile abdestilliert werden. Die erfindungsgemäßen Urethane stellen wachsartige, gelb- bis braungefärbte Produkte dar.

Die erfindungsgemäßen Urethane stellen überraschend gute Textilbehandlungsmittel dar. Sie verleihen den Textilien vor allem eine hervorragende Hydrophobie und Oleophobie.

Sie weisen ferner in einem hohen Ausmaß die Eigenschaft auf, den starken Beanspruchungen, denen die ausgerüsteten Textilien ausgesetzt werden, beispielsweise beim Verstrecken, Texturieren und insbesondere beim Färben und Waschen, ohne irgendeinen Verlust an Wirkung standzuhalten. Ein unerwarteter und besonders großer Vorteil der erfindungsgemäßen Verbindungen liegt darin, daß sie auch in üblichen Textilbehandlungspräparationen, beispielsweise in Spinnpräparationen, eingesetzt werden können und dabei ihre hervorragende Wirkung nicht verlieren.

Das Textilmaterial kann natürlicher und/oder synthetischer Natur sein. Es besteht vorzugsweise aus Polyamid, Polyester und/oder Polyacrylnitril, wobei Polyamid besonders bevorzugt ist. Das Textilmaterial kann in beliebiger Form vorliegen, so zum Beispiel als Faden, Faser, Garn, Flocke, Gewebe, Gestrick, Gewirk, Teppich oder Vlies. Die Auftragsmenge an erfindungsgemäßer Verbindung wird so gewählt, daß auf dem Textilmaterial 0,02 bis 1 Gew.-% Fluor, vorzugsweise 0,04 bis 0,4 Gew.-% Fluor, vorhanden sind, berechnet aus der Menge an Fluor in der erfindungsgemäßen Verbindung; Gewichtsprozente bezogen auf das behandelte Textilmaterial. Die Behandlung des Textilmaterials mit den erfindungsgemäßen Urethanen erfolgt in der Regel entweder über die oben erwähnten Textilbehandlungspräparationen, denen die erfindungsgemäßen Urethane einverleibt worden sind, oder mit Hilfe von Lösungen, Emulsionen oder Dispersionen, die aus den Urethanen eigens bereitet worden sind. In den Lösungen, Emulsionen oder Dispersionen beziehungsweise in den Textilbehandlungspräparationen liegen sie in der Regel in einer Konzentration von 5 bis 40 beziehungsweise 0,5 bis 5 Gew.-% vor, vorzugsweise 8 bis 30 beziehungsweise 1 bis 3 Gew.-%.

Die Behandlung der Textilien mit den genannten Lösungen, Emulsionen oder Dispersionen wird nach üblichen Methoden durchgeführt, so zum Beispiel durch Sprühen, Tauchen, Foulardieren und dergleichen. Anschließend wird das getränkte Textilmaterial getrocknet und einer Wärmebehandlung unterworfen. Die Wärmebehandlung wird in der Regel in der Weise durchgeführt, daß das Textilmaterial auf eine Temperatur von 130 bis 200 °C erhitzt und bei dieser Temperatur 10 Sekunden bis 10 Minuten lang gehalten wird. Das mit den erfindungsgemäßen Urethanen ausgerüstete Textilmaterial besitzt die oben erwähnten hervorragenden Eigenschaften. Die erfindungsgemäßen Verbindungen sind auch zur hydrophoben und oleophoben Ausrüstung von Leder hervorragend geeignet. Als Beispiele für Leder seien Rind-, Ziegen-, Schaf- und Schweineleder genannt. Die Auftragsmenge an erfindungsgemäßer Verbindung wird so gewählt, daß auf dem Leder 0,05 bis 1,5 Gew.-% Fluor, vorzugsweise 0,1 bis 1 Gew.-% Fluor, vorhanden sind, berechnet aus der Menge an Fluor in der Verbindung; Gewichtsprozente bezogen auf das behandelte Leder. Zum Aufbringen können die üblichen Arbeitsweisen zur Ausrüstung von Leder eingesetzt werden.

Die Erfindung wird nun anhand von Beispielen noch näher erläutert.

Erfindungsgemäße Verbindungen

Beispiel 1

In einem mit einem Rührer, Rückflußkühler, Thermometer, Tropftrichter und Heizbad ausgestatteten Glaskolben wurden 8 kg (15,7 mol) von einem im Handel erhältlichen Perfluoralkylethanol-Gemisch mit Perfluoralkyl = $C_8F_{17}$-$C_{16}F_{33}$ (OH-Zahl = 106), 8 kg 1,2,2-Trifluortrichlorethan ($CFCl_2$-$CF_2Cl$; Kp = 48 °C) als Lösungsmittel und 50 g Bortrifluoriddiethyletherat als Katalysator (das sind 0,6 Gew.-% Katalysator, bezogen auf Perfluoralkylethanol) vorgelegt. Zu dieser Lösung wurden bei 45 °C 2,9 kg (31,4 mol) Epichlorhydrin zugetropft, worauf 3 Stunden lang bei der Siedetemperatur des Lösungsmittels gehalten wurde. Anschließend wurde das eingesetzte Lösungsmittel im Vakuum (Wasserstrahlvakuum) abdestilliert. Es lag ein wachsartiges gelbgefärbtes Produkt vor. In dem so erhaltenen aliphatischen Fluoralkohol ist das Molverhältnis von Perfluoralkylethanol zu Epichlorhydrin = 1 : 2 (y in Formel 1 hat den Wert 2, der sich durch Mittelwertbildung von 1 bis 8 angelagerten Epichlorhydrin-Einheiten ergibt).

Die Umsetzung des alipahtischen Fluoralkohols mit Isocyanat zum Fluoralkohol-Isocyanat-Addukt (Addukt 1) erfolgte in einem mit Rührer, Rückflußkühler mit Trockenrohr, Thermometer und Heizbad ausgestatteten

Glaskolben.

Es wurden 85,0 g (0,13 mol) vom aliphatischen Fluoralkohol und 35,7 g (0,065 mol) Triisocyanat entsprechend Formel 3, und zwar ein als Handelsprodukt erhältliches Gemisch mit dem Triisocyanat gemäß Formel 3 als Hauptbestandteil, vorgelegt (das ist ein Molverhältnis von 2 : 1) und 4 Stunden lang bei 110 °C unter Rühren gehalten. Zur Mischung wurden nun 5 Tropfen Dibutylzinndilaurat dazugegeben, worauf sie zur Nachreaktion 3 Stunden lang bei 110 °C unter Rühren gehalten wurde. Das erhaltene Fluoralkohol-Isocyanat-Addukt (Addukt 1) war ein wachsartiges, gelbgefärbtes Produkt.

Die Umsetzung des Adduktes 1 mit den erfindungsgemäß einzusetzenden substituierten Phenolen oder Anilinen erfolgte ebenfalls in einem mit Rührer, Rückflußkühler mit Trockenrohr, Thermometer und Heizbad ausgestatteten Glaskolben.

Es wurden 100,0 g (62,6 mmol) vom Addukt 1 und 15,1 g (62,8 mmol) para-Hydroxystyrol vorgelegt (das ist ein Molverhältnis von 1 : 1) und 35 Stunden lang bei 110 °C unter Rühren gehalten. Es wurden 112 g, das sind 97 Gew.-% der Theorie, erfindungsgemäße Verbindung in Form eines wachsartigen, braungefärbten Produktes erhalten. Die Bruttozusammensetzung der erfindungsgemäßen Verbindung mit einem aliphatischen Fluoralkohol, Isocyanat und para-Hydroxystyrol im Molekül (Molverhältnis 2 : 1 : 1) entspricht der in der Tabelle nach den Beispielen angegebenen Formel B1.

Beispiel 2

Es wurden 231,4 g (0,12 mol) vom Addukt 1 des Beispiels 1 und 47,5 g (0,12 mol) Styrylphenol (das ist ein Molverhältnis von 1 : 1) in den oben angegebenen Glaskolben vorgelegt und 12 Stunden lang bei 110 °C unter Rühren gehalten. Zur Mischung wurden nun 5 Tropfen Dibutylzinndilaurat dazugegeben, worauf sie zur Nachreaktion 4 Stunden lang bei 110 °C unter Rühren gehalten wurde. Es wurden 277,8 g , das sind 99,6 Gew.-% der Theorie, erfindungsgemäße Verbindung in Form eines wachsartigen, braungefärbten Produktes erhalten. Die Bruttozusammensetzung der erfindungsgemäßen Verbindung mit einem aliphatischen Fluoralkohol, Isocyanat und Styrylphenol im Molekül (Molverhältnis 2 : 1 : 1) entspricht der in der genannten Tabelle angegebenen Formel B2.

Beispiel 3

Zur Herstellung eines weiteren Adduktes 1 wurden in dem in Beispiel 1 angegebenen Glaskolben 162,0 g (0,32 mol) vom Perfluoralkylethanol des Beispiels 1, 216,0 g (0,32 mol) vom aliphatischen Fluoralkohol des Beispiels 1 und 181,3 g (0,32 mol) des Triisocyanats vom Beispiel 1 vorgelegt (das ist ein Molverhältnis von 1 : 1 : 1) und 3 Stunden lang bei 110 °C unter Rühren gehalten; nach Zugabe von 9 Tropfen Dibutylzinndilaurat zur Mischung wurde diese weitere 3 Stunden lang bei 110 °C gerührt. Das erhaltene Fluoralkohol-Isocyanat-Addukt (Addukt 1) war ein wachsartiges, gelbgefärbtes Produkt. Zur erfindungsgemäßen Umsetzung dieses Adduktes 1 wurden analog Beispiel 1 559,0 g(0,32 mol) von dem Addukt und 83,8 g (0,32 mol) para-Dodecylphenol vorgelegt (das ist ein Molverhältnis von 1 : 1) und 5 Stunden lang bei 110 °C unter Rühren gehalten. Es wurden 638 g, das sind 99 Gew.-% der Theorie, erfindungsgemäße Verbindung in Form eines wachsartigen, braungefärbten Produktes erhalten. Die Bruttozusammensetzung der erfindungsgemäßen Verbindung mit einem aliphatischen Fluoralkohol, Isocyanat und para-Dodecylphenol im Molekül (Molverhältnis 2 : 1 : 1) entspricht der Formel B3.

Beispiel 4

Ansatz:
289,3 g (0,15 mol) vom Addukt 1 des Beispiels 3.
22,5 g (0,15 mol) para-tert.-Butylphenol.
Durchführung wie in Beispiel 3.
Ausbeute: 304 g, das sind 97,5 Gew.-% der Theorie, an erfindungsgemäßer Verbindung in Form eines wachsartigen, braungefärbten Produktes. Die Bruttozusammensetzung der erfindungsgemäßen Verbindung mit einem aliphatischen Fluoralkohol, Isocyanat und para-tert.-Butylphenol im Molekül (Molverhältnis 2 : 1 : 1) entspricht der Formel B4.

Beispiel 5

Ansatz:
289,3 g (0,15 mol) vom Addukt 1 des Beispiels 3.

46,1 g (0,15 mol) Tris-(para-Hydroxyphenylethan).
Durchführung wie in Beispiel 3.
Ausbeute: 330 g, das sind 98,5 Gew.-% der Theorie, an erfindungsgemäßer Verbindung in Form eines wachsartigen braungefärbten Produktes. Die Bruttozusammensetzung der erfindungsgemäßen Verbindung mit einem aliphatischen Fluoralkohol, Isocyanat und Tris-(para-Hydroxyphenylethan) im Molekül (Molverhältnis 2 : 1 : 1) entspricht der Formel B5.

T a b e l l e

| Nr. | Chemische Formeln der erfindungsgemäßen Verbindungen nach den Beispielen 1 bis 5 |
|---|---|
| B1 | 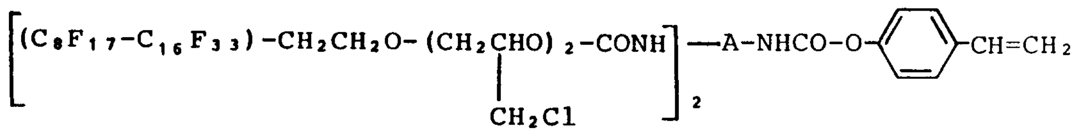 |
| B2 | 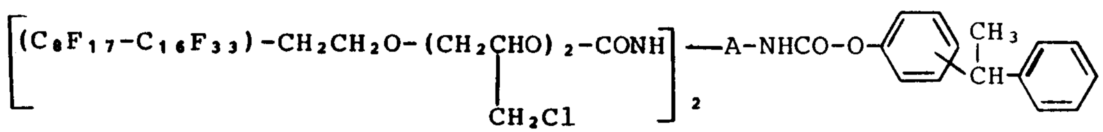 |
| B3 | 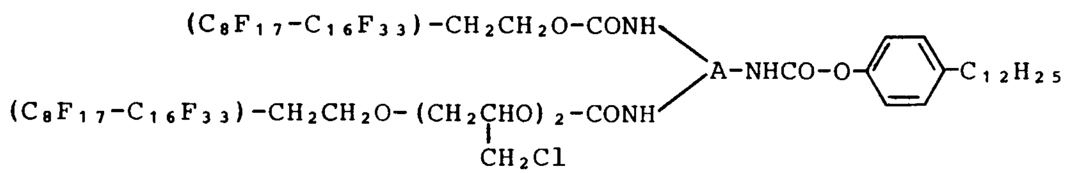 |
| B4 | 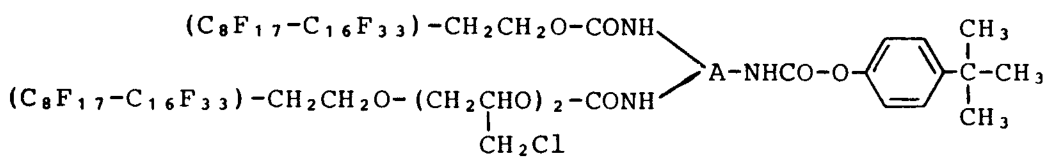 |
| B5 | 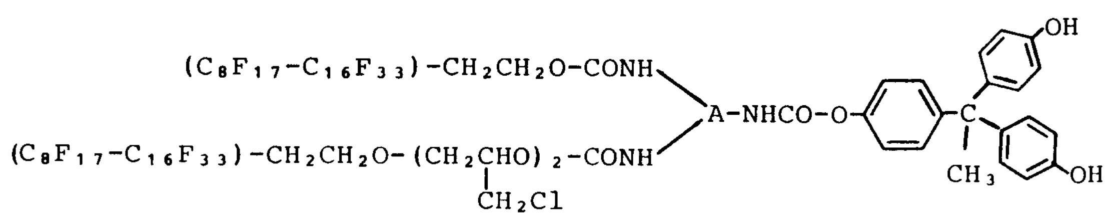 |

In den Formeln B1 bis B5 steht A für

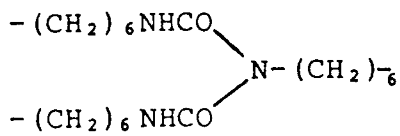

(vgl. obengenannte Formel 3).

Verwendung der erfindungsgemäßen Verbindungen

Beispiele I bis V

In den Beispielen I bis V wurden die erfindungsgemäßen Verbindungen B1 bis B5 mit Hilfe einer üblichen Spinnpräparation für Polyamidfasern getestet, die jeweils ca. 150 g erfindungsgemäße Verbindung pro 1000 g Spinnpräparation enthielt (die Spinnpräparation bestand also aus Wasser als Hauptkomponente, den üblichen ethoxylierten Fettalkoholen und langkettigen Aminoxiden als Präparationsmittel und ca. 15 Gew.-% erfindungsgemäßer Verbindung). Mit jeder der fünf Spinnpräparationen wurden jeweils gleiche Polyamid-6-Filamente behandelt, um soviel von der erfindungsgemäßen Verbindung und dem Präparationsmittel auf die Filamente aufzubringen, daß 0,08 Gew.-% Fluor und 1 Gew.-% Präparationsmittel auf den Filamenten vorlagen, Gewichtsprozente jeweils bezogen auf das Gewicht der Filamente. Dazu wurden die Filamente in üblicher Weise durch die Spinnpräparation gezogen, getrocknet und 30 Sekunden lang bei einer Temperatur von 200 °C gehalten (Wärmebehandlung, Kondensation). Aus den so behandelten Filamenten wurde jeweils ein Gewebe hergestellt. Es lagen fünf Gewebe mit den erfindungsgemäßen Verbindungen B1 bis B5 vor, wobei auf jedem Gewebe eine Fluorauflage von 0,08 Gew.-% und eine Präparationsmittelauflage von 1 Gew.-% vorhanden waren, Gewichtsprozente jeweils bezogen auf das Gewicht des Gewebes.
An den fünf Geweben wurden die Ölabweisung (Oleophobie) nach der AATCC-Prüfnorm 118 - 1966 und die Wasserabweisung (Hydrophobie) nach DIN 53 888 - 1965 geprüft, und zwar nach der beschriebenen Kondensation und nach einer dreistündigen Behandlung der kondensierten Gewebe mit einer alkalischen Kochwäsche. Bei dieser Behandlung wurden die einzelnen Gewebe in üblicher Weise 3 Stunden lang in einer alkalischen Waschflüssigkeit gekocht und anschließend getrocknet; die Waschflüssigkeit bestand aus 1 l Wasser, 1 g Trinatriumphosphat und 2 g eines Fettsäurepolyglykolesters, der durch Oxethylierung von Butandiol-1,4 mit 15 mol Ethylenoxid und anschließender Veresterung des Oxethylats mit 1 mol Ölsäure erhalten worden ist.
Die Ergebnisse aus den Beispielen I bis V sind nachstehend zusammengefaßt:

| Beispiele und getestete Verbindungen | Ölabweisung | | Wasserabweisung | |
|---|---|---|---|---|
| | nach der Kondensation | nach der Kochwäsche | nach der Kondensation | nach der Kochwäsche |
| I /B1 | 6 | 4 | 5 | 4 |
| II /B2 | 6 | 5 | 5 | 4 |
| III/B3 | 6 | 5 | 5 | 4 |
| IV /B4 | 6 | 5 | 5 | 4 |
| V /B5 | 5 | 4 | 4 | 4 |

Im folgenden wird der AATCC-Test 118 - 1966 (American Association of Textile Chemists and Colorists) und DIN 53 888 - 1965 (Deutsche Industrie-Norm) beschrieben:
Zur Bestimmung des Ölabweisungswertes nach AATCC-Test 118 - 1966 werden bekanntlich drei Tropfen von einer bestimmten Testflüssigkeit (siehe unten) vorsichtig auf das zu prüfende Textilmaterial aufgelegt. Einwirkungszeit: 30 Sekunden. Es wird der Wert angegeben, bei dem noch keine augenscheinliche Benetzung des Gewebes unter den Tropfen (nach abgelaufener Einwirkungszeit) hervorgerufen worden ist:

| Testflüssigkeit | Öblabweisungswert |
|---|---|
| Paraffinöl | 1 |
| Paraffinöl : n-Hexadecan = 65 : 35 | 2 |
| n-Hexadecan | 3 |
| n-Tetradecan | 4 |
| n-Dodecan | 5 |
| n-Decan | 6 |
| n-Octan | 7 |
| n-Heptan | 8 |

Ein Ölabweisungswert von 1 bedeutet den schlechtesten und ein Ölabweisungswert von 8 den besten Abweisungseffekt.

Zur Bestimmung des Wasserabweisungswertes nach DIN 53 888 - 1965 werden bekanntlich die zu prüfenden Textilien unter standardisierten Bedingungen beregnet, wobei gleichzeitig die Unterseite der Textilprobe mechanisch gerieben wird. Es wird der Wasserabperleffekt visuell mit den Noten 1 bis 5 beurteilt, wobei Note 1 den schlechtesten und Note 5 den besten Abperleffekt bedeutet.

Die Testergebnisse zeigen,daß mit den erfindungsgemäßen Urethanen eine sehr hohe Öl- und Wasserabweisung erreicht wird, und daß die erfindungsgemäßen Urethane auch Textilbehandlungspräparationen zugesetzt werden können.

**Patentansprüche**

**1.** Urethane aus aliphatischen Fluoralkoholen, Isocyanaten und substituierten aromatischen Verbindungen der nachstehenden allgemeinen Formel 1

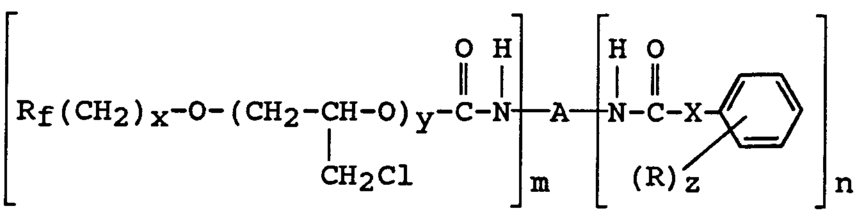

(1)

worin bedeuten:

- $R_f$ eine Perfluoralkylgruppe mit 4 bis 20 C-Atomen oder eine $R'_fSO_2NR^1$-Gruppe, in der $R'_f$ eine Perfluoralkylgruppe mit 4 bis 20 C-Atomen und $R^1$ H oder eine Alkylgruppe mit 1 bis 4 C-Atomen ist,
- x eine ganze Zahl von 1 bis 4,
- y eine Zahl von 0 bis 10,
- m eine Zahl von 1 bis 2 und
- n eine Zahl von 1 bis 2, wobei die Summe aus m + n höchstens 3 ist,
- A eine Gruppe entsprechend den nachstehenden Formeln 2 und 3:

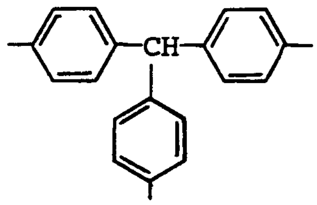

(2)

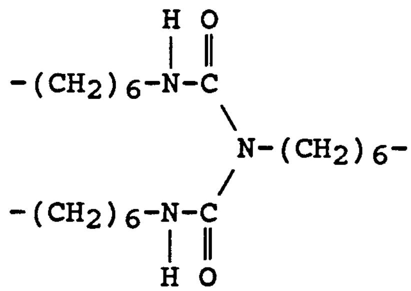

(3)

X -O- oder -NH-,

z 1, 2 oder 3, und

R eine Alkylgruppe mit 1 bis 18 C-Atomen, eine Alkenylgruppe mit 2 bis 18 C-Atomen, eine Arylgruppe oder eine Alkarylgruppe, wobei R auch mehrere dieser Bedeutungen haben kann.

**2.** Urethane nach Anspruch 1, dadurch gekennzeichnet, daß $R_f$ eine Perfluoralkylgruppe mit 6 bis 16 C-Atomen bedeutet, x = 2 und y = 1 bis 5 sind, m = 1 bis 2 und n = 1 bis 2 sind, wobei die Summe aus m + n höchstens 3 ist, A eine Gruppe entsprechend der Formel 3 bedeutet, z = 1 oder 2 ist und R eine Alkylgruppe mit 1 bis 12 C-Atomen bedeutet, eine Alkenylgruppe mit 2 bis 12 C-Atomen, eine Arylgruppe in Form von Phenyl oder Phenyl, das ein- bis dreifach mit $C_1$ bis $C_6$-Alkyl substituiert ist, oder eine Alkarylgruppe in Form einer Alkylgruppe mit 1 bis 4 C-Atomen, die mit 2 bis 3 Hydroxyphenylgruppen substituiert ist oder ein- bis dreifach substituiert ist mit Phenyl oder Phenyl, das ein- bis dreifach mit $C_1$ bis $C_6$-Alkyl substituiert ist.

**3.** Verfahren zur Herstellung der Urethane nach Anspruch 1, gekennzeichnet durch Reaktion eines aliphatischen Fluoralkohols der Formel

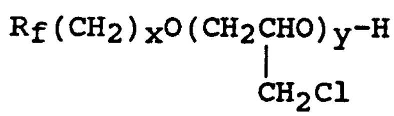

worin $R_f$, x und y die genannte Bedeutung haben,
mit einem Triisocyanat entsprechend einer der Gruppen der Formeln 2 und 3 zum Addukt der Formel

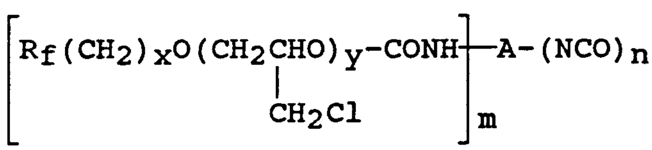

worin $R_f$, x, y, m, n und A die genannte Bedeutung haben,
und durch Reaktion dieses Adduktes mit einem substituierten Phenol oder Anilin der nachstehenden Formel

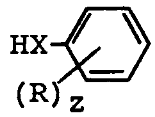

worin X, R und z die genannte Bedeutung haben,
zu den Urethanen der angegebenen Formel 1.

**4.** Verwendung der Urethane nach Anspruch 1 oder 2 zur Oleophob- und Hydrophob-Ausrüstung von Textilien und Leder.

**Claims**

**1.** Urethanes made from aliphatic fluorinated alcohols, isocyanates and substituted aromatic compounds of the formula 1 below

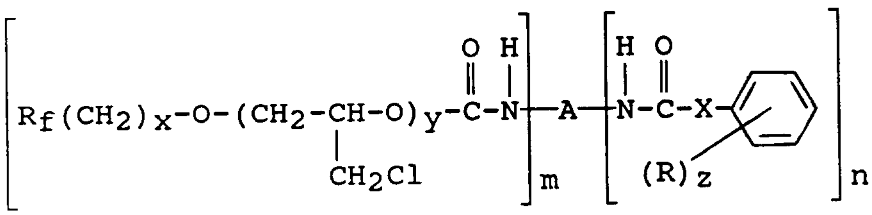

(1)

in which:

- $R_f$ denotes a perfluoroalkyl group having 4 to 20 carbon atoms or an $R'_fSO_2NR^1$ group, in which $R'_f$ is a perfluoroalkyl group having 4 to 20 carbon atoms and $R^1$ is H or an alkyl group having 1 to 4 carbon atoms,
- x denotes an integer from 1 to 4,
- y denotes a number from 0 to 10,
- m denotes a number from 1 to 2, and
- n denotes a number from 1 to 2, where the sum of m + n is at most 3,
- A denotes one of the groups conforming to the formulae 2 and 3 below:

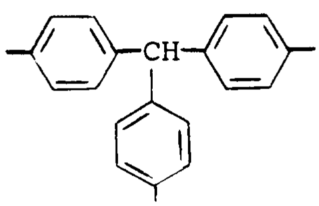

(2)

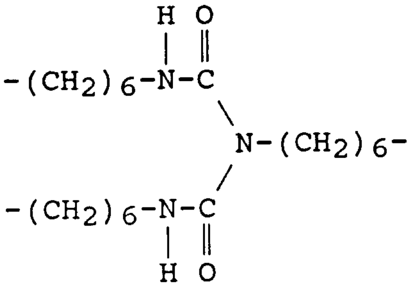

(3)

- X denotes -O- or -NH-,

z denotes 1, 2 or 3, and

R denotes an alkyl group having 1 to 18 carbon atoms, an alkenyl group having 2 to 18 carbon atoms, an aryl group or an alkaryl group, it also being possible for R to have several of these meanings.

**2.** Urethanes as claimed in claim 1, wherein $R_f$ denotes a perfluoroalkyl group having 6 to 16 carbon atoms, $x = 2$ and $y = 1$ to 5, $m = 1$ to 2 and $n = 1$ to 2, where the sum of $m + n$ is at most 3, A denotes a group conforming to the formula 3, $z = 1$ or 2, and R denotes an alkyl group having 1 to 12 carbon atoms, an alkenyl group having 2 to 12 carbon atoms, an aryl group in the form of phenyl or phenyl which is monosubstituted to trisubstituted by $C_1$ to $C_6$-alkyl, or an alkaryl group in the form of an alkyl group having 1 to 4 carbon atoms which is substituted by 2 to 3 hydroxyphenyl groups or is monosubstituted to trisubstituted by phenyl or phenyl which is monosubstituted to trisubstituted by $C_1$ to $C_6$-alkyl.

**3.** A process for the preparation of urethanes as claimed in claim 1, which comprises reacting an aliphatic fluorinated alcohol of the formula

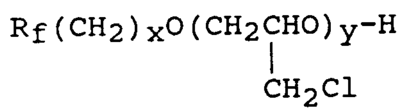

in which $R_f$, x and y are as defined above,
with a triisocyanate conforming to one of the groups of formulae 2 and 3, to form an adduct of the formula

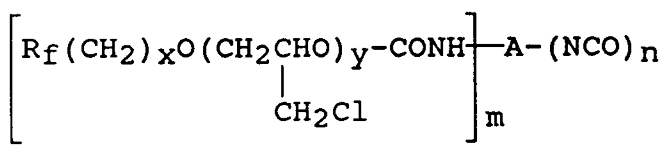

in which $R_f$, x, y, m, n and A are as defined above,
and reacting this adduct with a substituted phenol or aniline of the formula below

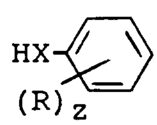

in which X, R and z are as defined above,
to form a urethane of the formula 1 indicated.

**4.** The use of the urethanes as claimed in claim 1 or 2, for oleophobic and hydrophobic finishing of textiles and leather.

## Revendications

**1.** Uréthannes obtenus à partir d'alcools fluorés aliphatiques d'isocyanates et de composés organiques substitués ayant la formule générale 1 ci-après

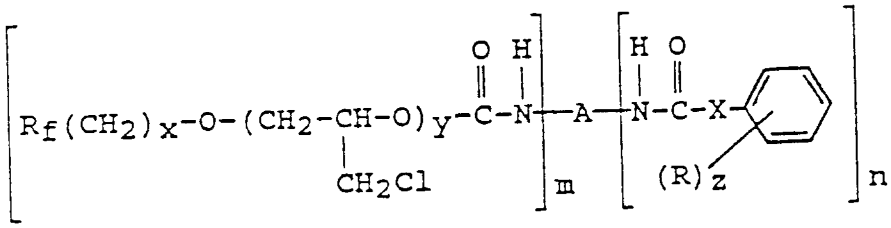

(1)

dans laquelle :

$R_f$ est un groupe perfluoralkyle ayant de 4 à 20 atomes de carbone ou un groupe $R'_fSO_2NR^1$ dans lequel $R'_f$ est un groupe perfluoralkyle ayant de 4 à 20 atomes de carbone et $R^1$ est H ou un groupe alkyle ayant de 1 à 4 atomes de carbone,

x est un nombre entier de 1 à 4,

y est un nombre de 0 à 10,

m est un nombre de 1 à 2, et

n est un nombre de 1 à 2, la somme m + n valant au plus 3,

A est un groupe selon les formules 2 et 3 ci-après :

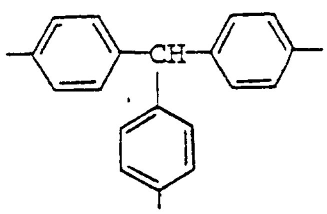

(2)

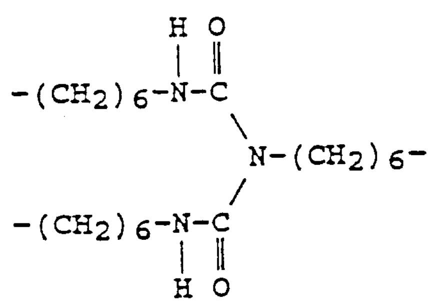

(3)

X est -O- ou -NH-,

z vaut 1, 2 ou 3, et

R est un groupe alkyle ayant de 1 à 18 atomes de carbone, un groupe alcényle ayant de 2 à 18 atomes de carbone, un groupe aryle ou un groupe aralkyle, R pouvant aussi avoir plusieurs de ces significations.

**2.** Uréthannes selon la revendication 1, caractérisés en ce que $R_f$ est un groupe perfluoralkyle ayant de 6 à 16 atomes de carbone, x vaut 2 et y vaut de 1 à 5, m vaut de 1 à 2 et n vaut de 1 à 2, la somme m + n étant au plus égale à 3, A est un groupe correspondant à la formule 3, z vaut 1 ou 2, et R est un groupe alkyle ayant de 1 à 12 atomes de carbone, un groupe alcényle ayant de 2 à 12 atomes de carbone, un groupe aryle sous forme des groupes phényle ou phényle une à trois fois substitués par des groupements alkyle en $C_1$ à $C_6$, ou encore un groupe aralkyle sous forme d'un groupe alkyle ayant de 1 à 4 atomes de carbone substitués par deux ou trois groupes hydroxyphényle et une à trois fois substitués par des radicaux phényle éventuellement une à trois fois substitués par des radicaux alkyle en $C_1$ à $C_6$.

**3.** Procédé pour préparer les uréthannes selon la revendication 1, caractérisé en ce qu'il consiste à faire réagir un alcool fluoré aliphatique de formule

$$R_f(CH2)_xO(CH_2\underset{\displaystyle CH_2Cl}{CHO})_y\text{-}H$$

dans laquelle $R_f$, x et y ont les significations données ci-dessus, avec un triisocyanate correspondant à l'un des groupes de formules 2 et 3, pour obtenir le produit d'addition de formule

$$\left[R_f(CH_2)_xO(CH_2\underset{\displaystyle CH_2Cl}{CHO})_y\text{-}CONH\right]_m\text{-}A\text{-}(NCO)_n$$

dans laquelle $R_f$, x, y, m, n et A ont les significations données ci-dessus, et à faire réagir ce produit d'addition avec un phénol ou une aniline substitués ayant la formule suivante :

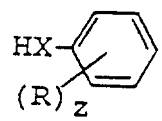

dans laquelle X, R et z ont les significations données ci-dessus, pour obtenir les uréthannes ayant la formule 1 indiquée.

**4.** Utilisation des uréthannes selon les revendications 1 ou 2 pour réaliser un apprêt oléophobe et hydrophobe sur des textiles et du cuir.